# EUROPEAN PATENT SPECIFICATION

(11) **EP 1 569 697 B1**
(45) Date of publication and mention of the grant of the patent: **12.11.2008**
(21) Application number: 03767966.9
(22) Date of filing: 08.12.2003
(51) Int. Cl.: A61L 15/18, A61L 15/42, A61P 17/02

(54) **WOUND DRESSINGS COMPRISING VANADATE COMPLEXED WITH ORGANIC LIGANDS**
WUNDVERBAND ENTHALTEND KOMPLEXE VON VANADAT UND ORGANISCHE VERBINDUNGEN
PANSEMENTS CONTENANT DU VANADATE COMPLEXE AVEC DES LIGANDS ORGANIQUES

(30) Priority: 12.12.2002 GB 0229012
(43) Date of publication of application: 07.09.2005
(73) Proprietor: Johnson & Johnson Medical Ltd., Edinburgh EH2 4NH (GB)
(72) Inventor: TROTTER, Patrick, Leeds LS6 4RD (GB)
(74) Representative: James, Anthony Christopher W.P.
(86) International application number: PCT/GB2003/005336
(87) International publication number: WO 2004/052412

(56) References cited:
- WO-A-97/47296
- US-A- 5 300 496
- US-A- 5 741 777

## Description

The present invention relates to wound dressings for use in wound healing.

Growth factors such as epidermal growth factor (EGF), platelet derived growth factor (PDGF) and fibroblast growth factor (FGF) play an important role in wound healing. These molecules act by binding to receptors on the cell surface. This binding induces conformational changes in the growth factor receptors, which in turn activates their intrinsic kinase activity and causes the phosphorylation of many intracellular proteins, including phospholipase C-g and PI-3 kinase.

In cells involved in wound healing (fibroblasts, keratinocytes, endothelial cells etc.) it is this signal transduction pathway that causes an increase in cell proliferation, the deposition of components of the extracellular matrix and is the molecular basis of the wound healing process.

Protein tyrosine phosphatases (PTP) are proteins that reverse the effects of protein kinases by a process called dephosphorylation. These proteins act as molecular switches that can switch off activation signals. Indeed the non-specific, cell permeable protein tyrosine phosphatase inhibitor vanadate has been shown to cause bone cell proliferation and bone collagen synthesis in *in vitro* studies (Lau, K.H. Tanimoto, H. and Baylink, D.K. (1988) Vanadate stimulates cell proliferation and bone collagen synthesis in vitro. Endocrinology. 123, 2858-67). In addition in capillary endothelial cells vanadate has been shown to mimic the effects of fibroblast growth factor in that it stimulates endothelial cells to invade collagen matrices and organise into tubules (Montesano, R., Pepper, M.S., Belin, D., Vassalli, J.D., and Orci, L. describe induction of angiogenesis in vitro by vanadate, an inhibitor of phosphotyrosine phosphatases (1988) J. Cell. Physiol. 134: 460-6).

US 5,741,777 (to Grinnell et al.) describes the modulation of wound contraction by blocking protein tyrosine phosphatase using tyrosine phosphatase inhibitors in the presence of a growth factor. No mention of increased cell proliferation is made and moreover US 5,741,777 employs a non-specific protein phosphatase (pervanadate). US 5,741,777 also states that because of the involvement of tyrosine kinases and tyrosine phosphatases in the regulation of cell growth, inhibition of tyrosine phosphatases is likely to be a promising means of inhibiting cell growth in general.

US 20020019412 A1 (to Henrik Sune Anderson et al.) discloses various uses of inhibitors of PTPases, e.g. in treating diabetes. However, the use of PTPase inhibitors in wound dressings is not disclosed.

Ehrich, H.P., Keefer, K.A., Maish, G.O. 3rd, Myers, R.L. and Mackay, D.R. in Plast Reconstr. Surg. 107(2), 471-7 (2001) disclose that vanadate ingestion increases the gain in wound breaking strength and leads to better organised collagen fibres during healing of acute wounds in rats. Vanadate inhibits the activity of a number of enzymes, including tyrosine phosphatase. There is no suggestion topical application of the vanadate, for example as or in a wound dressing.

Vanadate and pervanadate are non-specific tyrosine phosphatase inhibitors. That is they inhibit all tyrosine phosphatase enzymes and are not selective. They therefore modify many cellular processes and offer no control over what signal transduction pathways are modified. For example, Vanadate (Sodium Orthovanadate) is a broad spectrum inhibitor of protein tyrosine phosphatases. It is also known to inhibit Na+/K+ ATPase, acid and alkalinephosphatases, phosphofructokinase, and adenylate cyclase. It is therefore not selective in its molecular target and as such the specific cellular response following addition of vanadate is complex and is difficult to predict.

Pervanadate is a derivative of vanadate and can be made by adding H₂O₂ to vanadate. Pervanadate is more potent than vanadate due to having a greater ability to migrate through the cell membrane into the cell. It is however also very non-specific and as such is toxic. The toxicity can be explained by its low specificity, it inhibits all tyrosine phosphatases as well as other essential enzymes such as Na+/K+ ATPase, acid and alkaline phosphatases, phosphofructokinase, and adenylate cyclase.

It is desired to devise tyrosine phosphatase inhibitors having higher specificity than orthovanadate or pervanadate that are likely to affect only the signal transduction pathways that would be activated by natural growth factors. Unwanted side effects are thus minimised.

We have now discovered that certain organic complexes of vanadate greatly increase the rate of cell proliferation, collagen deposition and ultimately the rate of wound healing if used in a clinical situation. The effect is significantly greater that that observed for vanadate alone. Furthermore, the organic nature of these complexes means that they can be tailored to provide the desired absence of toxicity and side effects.

Accordingly, a first aspect of the invention provides a wound dressing comprising a complex of vanadate with an organic ligand, wherein said complex stimulates collagen deposition and fibroblast proliferation, wherein the complex is as defined in claim 1 and wherein the wound dressing does not comprise a growth factor.

It will be appreciated that the vanadate complex will normally carry an overall ionic charge, normally a cationic charge, and that it will then be associated with a suitable pharmaceutically acceptable counterion such as sulfate, chloride, (hydrogen) phosphate, p-toluene sulfonate, acetate, trifluoroacetate, propionate, citrate, malonate, succinate, lactate, oxadate, tartarate, benzoate, or mixtures thereof. The uses and products of the present invention encompass all medically acceptable salts and isomers of the claimed vanadate complexes.

The or each vanadate complex employed in the present invention is:
(i) bpv(pic) (bisperoxo pyridine-2-carboxyoxovanadate),
(ii) DHMV (Bis (N,N dimethylhydroxamido) hydroxoxovanadate, or
(iii) bpv(bipy) (bisperoxo(bipyridine)oxovanadate)

The vanadate complexes mentioned above are hydrolysed in aqueous solutions after a few days. This should prevent long term toxicological effects.

As indicated in the Examples section below, not all vanadate complexes enhance collagen deposition and fibroblast definition. For example, we have found that bpV(Phen) (i.e. bisperoxo-(1,10-phenanthroline)-oxovanadate) does not enhance collagen deposition and fibroblast proliferation. Indeed, bpV (Phen) appeared to be an inhibitor of proliferation.

Preferably, the complexes used in the wound dressings of the invention exhibit greater enhancement of collagen deposition and/or fibroblast proliferation definition, as determined using the assay methods described in the Examples section below, than vanadate or pervanadate alone, respectively.

It is an advantage of the present invention that the tyrosine phosphatase inhibitors are generally not substrates for the proteases present in wound fluid. Accordingly, the action of the or each tyrosine phosphatase inhibitor should not be affected by high protease levels which could make other similar (growth factor) treatments ineffective, especially in chronic wounds.

In certain embodiments of the invention, at least two or more vanadate complexes are employed. Where at least two vanadate complexes are employed, the two or more vanadate complexes may act synergistically together.

Suitably, the complex of vanadate with an organic ligand, wherein the complex is as defined in claim 1 and wherein the complex stimulates collagen deposition and fibroblast proliferation, is incorporated in a medicament for the treatment of wounds, wherein the medicament is a wound dressing according to claim 1.

The medicament is a wound dressing according to the first aspect of the present invention.

The term "wound" refers broadly to injuries to the skin and subcutaneous tissue initiated in different ways (e.g., pressure sores from extended bed rest and wounds induced by trauma) and with varying characteristics. Wounds may be classified into one of four grades depending on the depth of the wound: i) Grade I: wounds limited to the epithelium; ii) Grade II: wounds extending into the dermis; iii) Grade III: wounds extending into the subcutaneous tissue; and iv) Grade IV (or full-thickness wounds): wounds wherein bones are exposed (e.g., a bony pressure point such as the greater trochanter or the sacrum). The term "partial thickness wound" refers to wounds that encompass Grades I-III; examples of partial thickness wounds include bum wounds, pressure sores, venous stasis ulcers, and diabetic ulcers. The term "deep wound" is meant to include both Grade III and Grade IV wounds. The present invention contemplates treating all wound types, including deep wounds and chronic wounds. The term "chronic wound" refers to a wound that has not healed within 30 days. The delay in healing may, for example, be caused by elevated levels of matrix metalloproteinases (MMP's). Typically, the wound is a chronic wound. Preferably, it is selected from the group consisting of venous ulcers, pressure sores and decubitis ulcers.

As discussed in the experimental section below, we have found that improved results are achieved in the absence of supplementary growth factors. Thus, supplementary growth factors are not employed in the wound dressings, medicaments and uses of the invention.

Hence, with regard to the method of treatment, it is preferred that the patient is not administered a growth factor under circumstances which could adversely affect the therapeutic effects of the vanadate complex(s) on wound healing.

By "administered a growth factor under circumstances which could adversely affect the therapeutic effects of the vanadate complex(s)", we include, for example, the use of a medicament or dressing comprising both a vanadate complex(s) and a growth factor. Similarly, we include the administration of a growth factor to the wound shortly before or after administration of the vanadate complex(s) where the time difference between administration of the growth factor and the vanadate complex(s) is such that the growth factor could adversely affect the therapeutic effect of the vanadate complex(s) on wound healing.

Similarly, the medicaments and wound dressings of the invention do not comprise a growth factor. It is preferred that a growth factor is not administered to the wound shortly before or after administration of the medicament / wound dressing where the time difference between administration of the growth factor and the medicament / wound dressing is such that the growth factor could adversely affect the therapeutic effect of the vanadate complex(s) in the medicament / wound dressing on wound healing.

The vanadate complexes employed in the present invention modulate the first part of the signal transduction pathway. Treatment with vanadate complexes is likely to be more effective than other pharmacological/biochemical treatments that would be expected to affect downstream processes.

The term "protein tyrosine phosphatase inhibitor" refers to compounds which would cause an increase in the phosphotyrosine content of cells by a mechanism involving inhibition of tyrosine phosphatase activity. There are a variety of assays which may be used to determine if a compound is a protein tyrosine phosphatase inhibitor involving radioactive labeled cells (with phosphorous 32 or phosphorous -33 or antibodies specific to phosphotyrosine).

The present invention may be used to provide a method of increasing the rate of wound healing, the method comprising administering to the patient a complex of vanadate with an organic ligand which stimulates collagen deposition and fibroblast proliferation, wherein the complex is as defined in claim 1.

These therapeutic preparations can be administered to mammals for veterinary use, such as with domestic animals, and clinical use in humans in a manner similar to other therapeutic agents. In general, the dosage required for therapeutic efficacy will vary according to the type of use and mode of administration, as well as the particularized requirements of individual hosts.

Such compositions are typically prepared as liquid solutions or suspensions, or in solid forms. Formulations for wound healing usually will include such normally employed additives such as binders, fillers, carriers, preservatives, stabilizing agents, emulsifiers, buffers and excipients as, for example, pharmaceutical grades of mannitol, lactose, starch, magnesium stearate, sodium saccharin, cellulose, magnesium carbonate, and the like. These compositions take the form of solutions, suspensions, tablets, pills, capsules, sustained release formulations, or powders, and typically contain 1%-95% of active ingredient, preferably 2%-70%.

The vanadate complex may be mixed with diluents or excipients which are physiological tolerable and compatible. Suitable diluents and excipients are, for example, water, saline, dextrose, glycerol, or the like, and combinations thereof. In addition, if desired the compositions may contain minor amounts of auxiliary substances such as wetting or emulsifying agents, stabilizing or pH buffering agents.

In a further aspect, the present invention provides the use of a complex of vanadate with an organic ligand wherein the complex is as defined in claim 1 for the preparation of a medicament for the treatment of wounds. The medicament is a wound dressing. Preferably, the format of the medicament is as described above. Preferably, the wound is a venous ulcer, a pressure sore or a diabetic ulcer.

Preferably, the complexes used in the further aspect of the invention induce enhanced fibroblast proliferation and/or collagen deposition rates as measured according to the procedures specified below, relative to a negative control, and more preferably when measured relative to orthovanadate as positive control.

It will be appreciated that any alternative or preferred feature or combination of features that is disclosed herein in relation to any one aspect of the invention is hereby disclosed in relation to any other aspect of the invention.

### BRIEF DESCRIPTION OF THE FIGURES

Figure 1 shows measured collagen deposition in the presence of a range of vanadate complexes with organic ligands, at different concentrations;
Figure 2 shows measured fibroblast cell proliferation for the vanadate complexes and concentrations of Figure 1; and
Figure 3 shows a comparison of measured fibroblast cell proliferation for vanadate at 0.1mmol and 0.01 mmol, with the same measurement made for bpv(pic) at 0.0084mmol.

### EXAMPLES

To investigate the effect of a range of tyrosine phosphatase inhibitors on cell proliferation and collagen deposition the following experiments were performed.

### Cell treatments

Adult dermal fibroblasts were grown until 95% confluent, trypsinized (2ml trypsin plate) to prepare a single suspension of fibroblasts. These cells were diluted in DMEM (+ 10 % FBS) media and diluted to a concentration of 25000 cells /ml. 100ml was placed in each well of a 96 well microplate (2500 cells /well). The fibroblasts were allowed to adhere and spread by incubating for 24hours (at 37°C, 5% CO₂ in a humidified atmosphere) at which point the media was removed and replaced with serum free DMEM +/- stimulant/sample.

The following vanadate complexes were added in quadruplicate: bpV(pic) was added to a final concentration of 8.14 and 0.81µM, bpV(bipy) (bisperoxo(bipyridine)oxovanadate) was added to a final concentration of 178 and 17.8µM. bpV(phen) was added to a final concentration of 12 or 0.124µM, DMHV (bis-dimethylhydroxamido vanadate, (Me₂NO)₂VO(OH)) was added to a final concentration of 6.4 or 64Mm. The vanadate complexes were all obtained as the solid potassium salts from Calbiochem, Catalog numbers 203705, 322130, and 203695.

Negative control samples were untreated and positive control samples were cells treated with DMEM containing 10% FBS or 0.15ng/ml epidermal growth factor. As a further positive control, Dephostatin (a known tyrosine protein phosphatase inhibitor obtained from Calbiochem, catalog number 263200) was added to a final concentration of 23.8µM.

Additional experiments were performed using the phosphatase inhibitors indicated above in the presence of epidermal growth factor (0.015ng/ml) or platelet derived growth factor (6ng/ml).

### XTT cell Proliferation assay

After 72hrs the fluid was aspirated off and 50µl of the XTT labelling mixture (prepared by adding 0.1ml Electron coupling reagent to every 5mls of XTT labelling reagent) was added to each well. The plates were read at 450nm, using a MR5000 plate reader at 2, 2.5 and 3.5 hrs. The results shown are the absorbances compared to the negative and positive controls at 3.5hrs.

### Determination of collagen levels

The amount of collagen produce and deposited into the extracellular environment under each experimental condition was measured using an ELISA method. Collagen type I standards (Sigma cat no 7774, lot 32K3775) and material aspirated from the fibroblasts were coated onto 96 well plates (FALCON 3072) by incubation at 37°C for 30min. Non-specific sites were blocked by the addition of 200ml of 5% solution of bovine serum albumin (BSA) for 30min. Each well was incubated with a monoclonal antibody against collagen used at a dilution of 1/2000 ((sigma cat no. 2456, lot 081K4897) overnight at 4°C.

Samples were washed five times for 5minutes in wash buffer (PBS+0.05%(v/v) TWEEN-20) and incubated with an HRP-conjugated second antibody against mouse IgG at a dilution of 1/10000 for 2hr. Each well was washed five times in wash buffer. Deposited collagen was detected using the Sigma FAST method used according to the manufacturers instructions. The plates were incubated at room temperature for 30minutes and read using a MR5000 plate reader at 450nm. The standard curve was linear at 0.05-2µg, and results are presented as µg/ml in the extracellular environment.

### Results

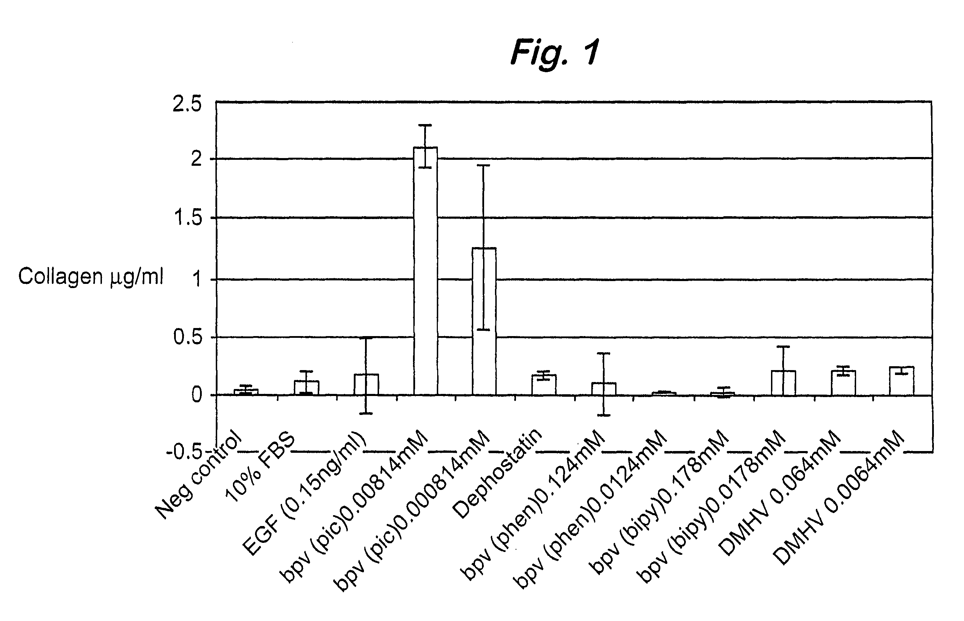

The graph in Figure 1 shows the amount of collagen deposited by fibroblasts in the presence of phosphatase inhibitors compared to non treated (negative control, far left), and foetal bovine serum (FBS) and EGF positive controls (2^{nd} and 3^{rd} left). BpV (pic) was the most effective activator of collagen deposition at 8.14µM. Dephostatin, (23.8µM), bpV (bipy) (17.8µM) and DMHV (6.4-64µM) demonstrated rates of collagen proliferation equivalent to the positive control.

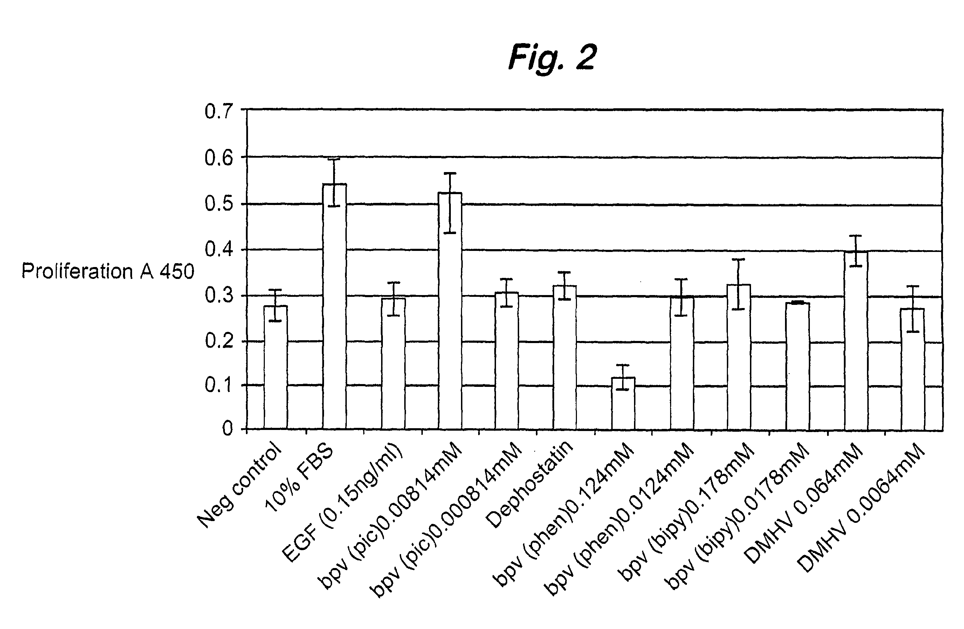

The graph in Figure 2 shows the amount of fibroblast proliferation in the presence of phosphatase inhibitors compared to non treated (negative control, far left), and a foetal bovine serum and EGF positive controls. BpV(pic) was the most effective activator cell proliferation at 8.14µM. This was similar to the 10% FBS positive control. Dephostatin, (23.8µM), bpV(bipy) (17.8µM) and DMHV (64µM) demonstrated rates of proliferation greater than the negative control.

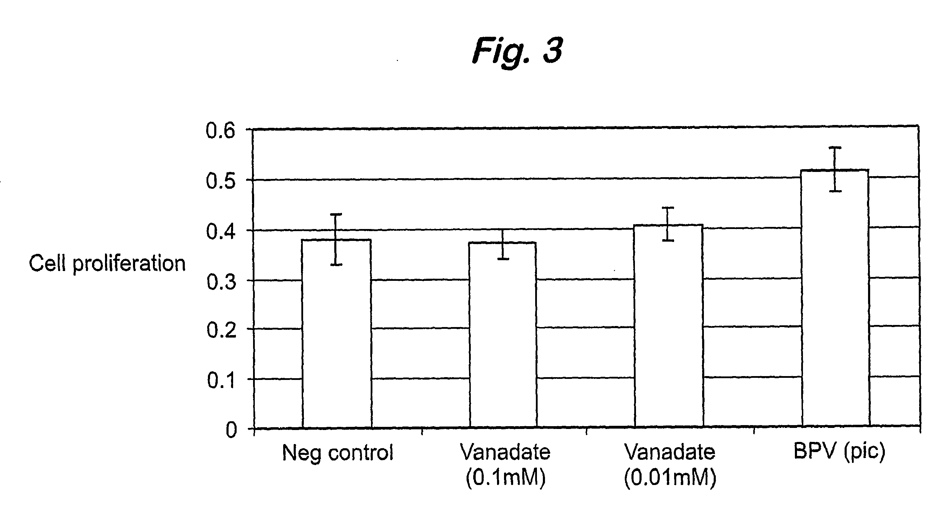

The graph in Figure 3 shows the amount of fibroblast proliferation in the presence of bpv(pic) compared to non treated (negative control, far left), and vanadate at 0.1 and 0.01mmolar (positive controls). BpV(pic) was the more effective activator of cell proliferation at 8.14µM.

From these studies all the compounds tested (with the exception of bpV (phen) showed some ability to stimulate cell proliferation and collagen deposition. Bpv (pic) appeared to be more effective than even the 10% FBS positive control, while DMHV appeared to be equivalent to the DMHV positive control.

The data above show stimulation of cell proliferation and collagen deposition in the absence of growth factors. Experiments were also performed in the presence and absence of epidermal growth factor (EGF (0.015ng/ml)) or platelet derived growth factor (6ng/ml). The results demonstrated that cell activation did occur did still occur in the presence of phosphatase inhibitors and growth factor, but that the amount of proliferation and collagen deposition was not significantly higher than with inhibitor alone.

## Claims

1. A wound dressing comprising a complex of vanadate with an organic ligand, wherein said complex stimulates collagen deposition and fibroblast proliferation and wherein the complex is bpv (pic) (Bis peroxo (5-hydroxy pyridine-2-carboxyvanadate)), bpv (bipy) (bisperoxo(bipyridine)oxovanadate) or DHMV (Bis (N,N dimethyl hydroxamido) hydroxoxovanadate), or a medically acceptable salt thereof, wherein the wound dressing does not comprise a growth factor.

2. Use of a complex of vanadate with an organic ligand, wherein the complex stimulates collagen deposition and fibroblast proliferation, in the manufacture of a medicament for the treatment of wounds, wherein the complex is bpv (pic) Bis peroxo (5-hydroxy pyridine-2-carboxyvanadate)), bpv (bipy) (bisperoxo(bipyridine)oxovanadate) or DHMV (Bis (N,N dimethyl hydroxamido) hydroxoxouvanadate), or a medically acceptable salt thereof, and wherein the medicament is a wound dressing according to claim 1.

3. Use according to claim 2, wherein the wound is selected from the group consisting of venous ulcer, pressure sore and decubitis ulcer.

## Patentansprüche

1. Wundverband, der einen Komplex von Vanadat mit einem organischen Liganden umfasst, wobei der Komplex Collagen-Abscheidung und Fibroblasten-Proliferation stimuliert und wobei der Komplex bpv (pic) (Bisperoxo(5-hydroxypyridin-2-carboxyvanadat)), bpv (bipy) (Bisperoxo(bipyridin)oxovanadat) oder DHMV (Bis(N,N-dimethylhydroxamido)hydroxoxovanadat)) oder ein medizinisch verträgliches Salz davon ist, wobei der Wundverband keinen Wachstumsfaktor umfasst.

2. Verwendung eines Komplexes von Vanadat mit einem organischen Liganden, wobei der Komplex Collagen-Abscheidung und Fibroblasten-Proliferation stimuliert, bei der Herstellung eines Arzneimittels zur Behandlung von Wunden, wobei der Komplex bpv (pic) (Bisperoxo(5-hydroxypyridin-2-carboxyvanadat)), bpv (bipy) (Bisperoxo(bipyridin)oxovanadat) oder DHMV (Bis(N,N-dimethylhydroxamido)hydroxoxovanadat)) oder ein medizinisch verträgliches Salz davon ist und wobei das Arzneimittel ein Wundverband nach Anspruch 1 ist.

3. Verwendung nach Anspruch 2, wobei die Wunde ausgewählt ist aus der Gruppe, bestehend aus venösem Geschwür, Druckbrand und Decubitusgeschwür.

## Revendications

1. Pansement comprenant un complexe de vanadate avec un ligand organique, ledit complexe stimulant le dépôt du collagène et la prolifération des fibroblastes, et le complexe étant le bpv (pic) (Bisperoxo(5-hydroxy-pyridine-2-carboxyvanadate)), le bpv (bipy) (bisperoxo(bipyridine)oxovanadate) ou le DHMV (bis-(N,N-diméthylhydroxamido)-hydroxo-oxovanadate) ou un sel médicalement acceptable de ceux-ci, le pansement ne comprenant pas de facteur de croissance.

2. Utilisation d'un complexe de vanadate avec un ligand organique, le complexe stimulant le dépôt du collagène et la prolifération des fibroblastes, dans la fabrication d'un médicament pour le traitement des plaies, le complexe étant le bpv (pic) (Bisperoxo (5-hydroxypyridine-2-carboxyvanadate)), le bpv (bipy) (bisperoxo(bipyridine)oxovanadate) ou le DHMV (bis(N,N-diméthylhydroxamido)hydroxo-oxovanadate) ou un sel médicalement acceptable de ceux-ci, et le médicament étant un pansement selon la revendication 1.

3. Utilisation selon la revendication 2, dans laquelle la plaie est choisie dans le groupe comprenant les ulcères variqueux, les escarres de pression et les escarres de décubitus.
